(19) Europäisches Patentamt
European Patent Office
Office européen des brevets

(11) Veröffentlichungsnummer: **0 026 856**
**B1**

(12) **EUROPÄISCHE PATENTSCHRIFT**

(45) Veröffentlichungstag der Patentschrift: **16.03.83**

(51) Int. Cl.³: **A 61 K 31/415** // C07D233/60, C07C49/255, C07C49/16, C07C49/167, C07C143/68

(21) Anmeldenummer: **80105458.6**

(22) Anmeldetag: **12.09.80**

(54) **Ein acyliertes Imidazolyl-gamma-fluorpinakolylderivat enthaltendes antimykotisches Mittel und dessen Herstellung.**

(30) Priorität: **24.09.79 DE 2938550**

(43) Veröffentlichungstag der Anmeldung: **15.04.81 Patentblatt 81/15**

(45) Bekanntmachung des Hinweises auf die Patenterteilung: **16.03.83 Patentblatt 83/11**

(84) Benannte Vertragsstaaten: **AT BE CH DE FR GB IT LI NL SE**

(56) Entgegenhaltungen:
**EP-A-0 019 130**
**DE-A-2 811 916**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/152, D-5600 Wuppertal 1 (DE)**
Erfinder: **Büchel, Karl Heinz, Dr. Prof., Haus an der Dachsdelle Dabringhausener Strasse 42, D-5093 Burscheid (DE)**
Erfinder: **Stetter, Jörg, Dr., Gellertweg 4, D-5600 Wuppertal 1 (DE)**
Erfinder: **Plempel, Manfred, Dr., Pahlkestrasse 5, D-5600 Wuppertal 1 (DE)**
Erfinder: **Haller, Ingo, Dr., Viktoriastrasse 99, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Ein acyliertes Imidazolyl-γ-fluorpinakolylderivat enthaltendes antimykotisches Mittel und dessen Herstellung

Die Erfindung betrifft ein antimykotisches Mittel, das durch einen Gehalt an mindestens einem acylierten Imidazolyl-γ-fluorpinakolylderivat der allgemeinen Formel I gekennzeichnet ist.

Es ist bereits bekannt geworden, dass acylierte 1-Imidazolyl-2-hydroxybutanderivate, wie insbesondere im Phenylteil substituierte 2-Acyloxy- bzw. 2-Carbamoyloxy-3,3-dimethyl-1-phenoxy-1-(imidazol-1-yl)butane, gute antimikrobielle Eigenschaften aufweisen (vgl. DE-OS Nr. 2604865). Deren Wirkung ist jedoch, insbesondere *in vivo* gegen *Candida* nicht immer ganz befriedigend.

Es wurde gefunden, dass die neuen acylierten Imidazolyl-γ-fluorpinakolylderivate der allgemeinen Formel I

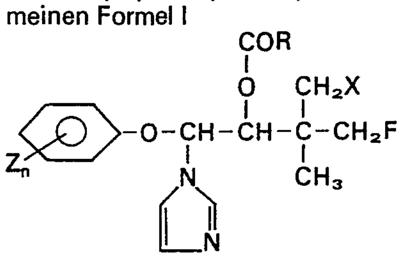

(I)

in welcher

R für Methyl, Äthyl, Isobutyl, Chlormethyl, Dichlormethyl, Chloräthyl, Chlorpropyl, Methacryl, Cyclohexyl, gegebenenfalls einfach oder mehrfach durch Chlor, Brom, Methyl oder Methoxy substituiertes Phenyl, Benzyl oder Phenoxymethyl, für Methoxy, Äthoxy, Isopropoxy, Butoxy oder Isobutoxy, Methyl- oder Äthylamino, Dimethylamino, Phenylamino, Chlorphenylamino, Chloräthylamino, Methoxycarbonylamino, Äthoxycarbonylamino oder Methoxymethylamino steht,

X für Wasserstoff oder Fluor steht,

Z für Chlor, Brom, Methyl, Äthyl, Cyclohexyl, Methoxy, Methylthio, Trifluormethyl, Methoxycarbonyl, Cyano, Nitro oder gegebenenfalls durch Chlor substituiertes Phenyl, Benzyl oder Phenoxy steht, und

n für 0, 1 oder 2 steht,

und deren physiologisch verträglichen Säureadditionssalze und Metallsalzkomplexe gute antimikrobielle, insbesondere antimykotische Eigenschaften aufweisen.

Die Verbindungen der allgemeinen Formel I besitzen zwei asymmetrische Kohlenstoffatome; sie können deshalb in der erythro- wie in der threo-Form vorliegen. In beiden Fällen liegen sie vorwiegend als Racemate vor.

Überraschenderweise zeigen die erfindungsgemäss verwendbaren acylierten Imidazolyl-γ-fluorpinakolylderivate der allgemeinen Formel I eine bessere antimykotische Wirksamkeit, insbesondere *in vivo* gegen *Candida*, als die aus dem Stand der Technik bekannten acylierten bzw. carbamoylierten 1-Imidazolyl-2-hydroxybutanderivate, die chemisch die naheliegendsten Verbindungen sind. Die erfindungsgemäss verwendbaren Stoffe stellen somit eine Bereicherung der Pharmazie dar.

Im einzelnen seien ausser den Herstellungsbeispielen und den Beispielen der Tabelle 1 folgende Verbindungen der allgemeinen Formel I genannt:

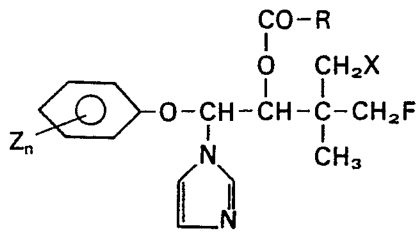

(I)

| R | X | $Z_n$ |
|---|---|---|
| $-CHCl_2$ | H | 4-Cl |
| $-CH_2Cl$ | H | 4-Cl |
| $-CH_3$ | H | 4-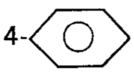 |
| -NH-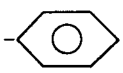 | H | 4-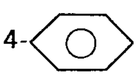 |
| -NH-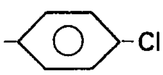 | H | 4-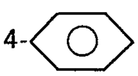 |
| $-NHCH_3$ | H | 4-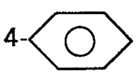 |
| $-NHCH_3$ | H | 4-Cl; 2-$CH_3$ |
| $-NHCH_3$ | H | 2-Cl |
| $-NHC_2H_3$ | H | 4-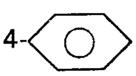 |
| $-C_4H_9$-i | H | 4-Br |
| $-CH_3$ | H | 2,4-$Cl_2$ |
| $-C_2H_5$ | H | 2,4-$Cl_2$ |
| $-CH_3$ | H | 4-$OCH_3$ |
| $-CH_2Cl$ | H | 3-$CF_3$ |
| $-CHXl_2$ | H | 4-CO-$OCH_3$ |
| $-NHCH_2OCH_3$ | H | 4-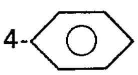 |
| $-NHCH_2OC_2H_5$ | H | 4-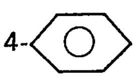 |
| $-NH-COOCH_3$ | H | 4-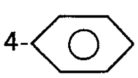 |
| $-NH-COOC_2H_5$ | H | 4-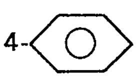 |
| $-N(CH_3)_2$ | H | 4-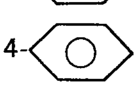 |
| $-OCH_3$ | H | 4-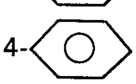 |
| $-CH_3$ | H | 4-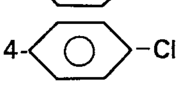 |

| R | X | $Z_n$ |
|---|---|---|
| $-CH_3$ | H | 4-O-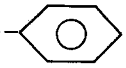 |
| $-CH_3$ | H | 4-O-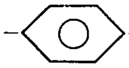-Cl |
| $-CH_3$ | H | 4-CN |
| $-CH_3$ | H | 4-$NO_2$ |
| $-CH_2OC_2H_5$ | H | 2,4-$Cl_2$ |
| $-NHCH(CH_3)_2$ | H | 2,4-$Cl_2$ |
| $-NH-CH_2OCH_3$ | H | 2,4-$Cl_2$ |
| $-OC_2H_5$ | H | 2,4-$Cl_2$ |
| $-C(CH_3)=CH_2$ | H | 2,4-$Cl_2$ |
| $-NH-CH_3$ | H | 2,4-$Cl_2$ |
| $-CH_2-CH(CH_3)_2$ | H | 2,4-$Cl_2$ |
| $-CH_2-$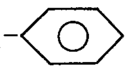 | H | 2,4-$Cl_2$ |
| $-CH_2-CH_2Cl$ | H | 2,4-$Cl_2$ |
| $-CH_2-CH_2-CH_2Cl$ | H | 2,4-$Cl_2$ |
| -NH-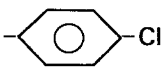-Cl | H | 2,4-$Cl_2$ |
| $-CHCl_2$ | H | 2,4-$Cl_2$ |
| $-CH_2Cl$ | H | 2,4-$Cl_2$ |
| 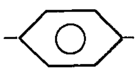-$OCH_3$ | H | 2,4-$Cl_2$ |
| $-CH_2-O-$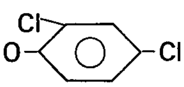-Cl | H | 2,4-$Cl_2$ |
| 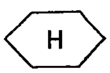 | H | 2,4-$Cl_2$ |
| 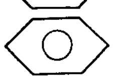 | H | 2,4-$Cl_2$ |
| 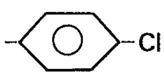-Cl | H | 2,4-$Cl_2$ |
| $-CH_2OC_2H_5$ | H | 4-Cl |
| $-NH-CH(CH_3)_2$ | H | 4-Cl |
| $-NH-CH_2OCH_3$ | H | 4-Cl |
| $-OC_2H_5$ | H | 4-Cl |
| $-C(CH_3)=CH_2$ | H | 4-Cl |
| $-CH_2-CH(CH_3)_2$ | H | 4-Cl |
| $-CH_2-$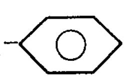 | H | 4-Cl |
| $-CH_2CH_2Cl$ | H | 4-Cl |
| $-CH_2-CH_2-CH_2Cl$ | H | 4-Cl |
| -NH-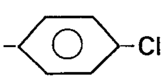-Cl | H | 4-Cl |
| 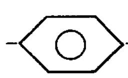-$OCH_3$ | H | 4-Cl |
| $-CH_2-O-$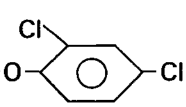-Cl | H | 4-Cl |
| $-NH-CH_3$ | H | 4-Cl |
| $-CH_3$ | H | 4-Cl |
| 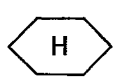 | H | 4-Cl |
| 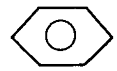 | H | 4-Cl |
| 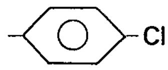-Cl | H | 4-Cl |
| $-NHCH_3$ | F | 2,4-$Cl_2$ |
| $-NHCH_3$ | F | 4-Cl |
| $-CH_3$ | F | 4-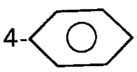-Cl |
| $-NHCH_3$ | F | 4-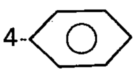 |
| $-NHC_2H_5$ | F | 4-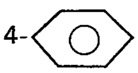 |
| $-NHCH_2OCH_3$ | F | 4-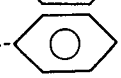 |
| $-NH-CH(CH_3)_2$ | F | 4-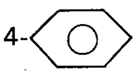 |
| $-CH_2OC_2H_5$ | F | 4-Cl |
| $-NH-CH(CH_3)_2$ | F | 4-Cl |
| $-NHCH_2OCH_3$ | F | 4-Cl |
| $-OC_2H_5$ | F | 4-Cl |
| $-C(CH_3)=CH_2$ | F | 4-Cl |
| $-CH_2-CH(CH_3)_2$ | F | 4-Cl |
| $-CH_2-$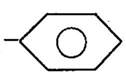 | F | 4-Cl |
| $-CH_2-CH_2Cl$ | F | 4-Cl |
| $-CH_2-CH_2-CH_2Cl$ | F | 4-Cl |
| -NH-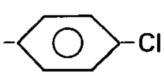-Cl | F | 4-Cl |
| $-CHCl_2$ | F | 4-Cl |
| $-CH_2Cl$ | F | 4-Cl |
| 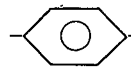-$OCH_3$ | F | 4-Cl |
| $-CH_2-O-$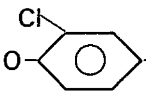-Cl | F | 4-Cl |
| 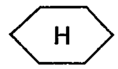 | F | 4-Cl |

| R | X | $Z_n$ |
|---|---|---|
| 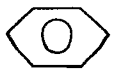 | F | 4-Cl |
| 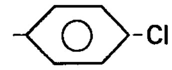 | F | 4-Cl |
| $-CH_2OC_2H_5$ | F | 2,4-$Cl_2$ |
| $-NH-CH(CH_3)_2$ | F | 2,4-$Cl_2$ |
| $-NH-CH_2OCH_3$ | F | 2,4-$Cl_2$ |
| $-OC_2H_5$ | F | 2,4-$Cl_2$ |
| $-C(CH_3)=CH_2$ | F | 2,4-$Cl_2$ |
| $-CH_2-CH(CH_3)_2$ | F | 2,4-$Cl_2$ |
| 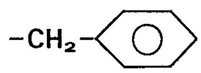 | F | 2,4-$Cl_2$ |
| $-CH_2-CH_2Cl$ | F | 2,4-$Cl_2$ |
| $-CH_2-CH_2-CH_2Cl$ | F | 2,4-$Cl_2$ |
| 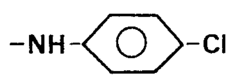 | F | 2,4-$Cl_2$ |
| $-CHCl_2$ | F | 2,4-$Cl_2$ |
| $-CH_2Cl$ | F | 2,4-$Cl_2$ |
| 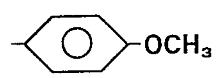 | F | 2,4-$Cl_2$ |
| 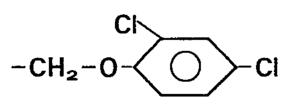 | F | 2,4-$Cl_2$ |
| 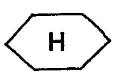 | F | 2,4-$Cl_2$ |
| 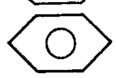 | F | 2,4-$Cl_2$ |
| 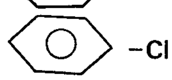 | F | 2,4-$Cl_2$ |

Die erfindungsgemäss zu verwendenden Wirkstoffe, deren Säureadditionssalze und Metallsalzkomplexe sind noch nicht bekannt. Sie können jedoch gemäss einem eigenen Vorschlag hergestellt werden, indem man 1-Imidazolyl-2-hydroxybutanderivate der allgemeinen Formel II

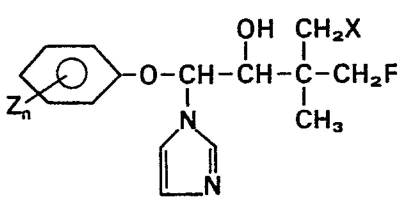 (II)

in welcher

X, Z und n die oben angegebene Bedeutung haben,

a) mit Säurehalogeniden nach bekannten Methoden, z.B. in molaren Mengen in Gegenwart eines inerten organischen Lösungsmittels, z.B. Essigester, bei Temperaturen zwischen 0 und 100° C umsetzt.

Die Verbindungen der allgemeinen Formel I fallen in Form ihrer Hydrohalogenide an und können als solche isoliert werden, indem man sie durch Zugabe eines organischen Selvens, z.B. Hexan, ausfällt, absaugt und gegebenenfalls durch Umkristallisation reinigt. Die Verbindungen der allgemeinen Formel I können auch in Form ihrer freien Basen isoliert werden, indem man das Reaktionsgemisch mit wässeriger Natriumhydrogencarbonatlösung versetzt und die Base nach üblichen Methoden isoliert.

Die Verbindungen der allgemeinen Formel I können ferner dadurch hergestellt werden, dass man Verbindungen der allgemeinen Formel II

b) mit Säureanhydriden nach bekannten Methoden, z.B. in molaren Mengen in Gegenwart eines inerten organischen Lösungsmittels, z.B. Aceton oder Überschuss an Säureanhydrid und in Gegenwart eines sauren oder basischen Katalysators, z.B. Natriumacetat, bei Temperaturen zwischen 0 und 150° C umsetzt und die Verbindungen der allgemeinen Formel I in üblicher Weise isoliert, oder

c) mit Ketenen nach bekannten Methoden, z.B. in molaren Mengen in Gegenwart eines inerten organischen Lösungsmittels, z.B. Essigester, und in Gegenwart eines sauren oder basischen Katalysators, z.B. Natriumacetat, bei Temperaturen zwischen −10 und 70° C umsetzt und die Verbindungen der allgemeinen Formel I in üblicher Weise isoliert, oder

d) mit Isocyanaten nach bekannten Methoden, z.B. in molaren Mengen in Gegenwart eines inerten organischen Lösungsmittels, z.B. Essigester, und in Gegenwart eines Katalysators, z.B. Triäthylamin, bei Temperaturen zwischen 0 und 100° C umsetzt und die Verbindungen der allgemeinen Formel I in üblicher Weise isoliert, und gegebenenfalls durch Umsetzen mit Säuren die Säureadditionssalze bzw. durch Reaktion mit Metallsalzen die Metallsalzkomplexe herstellt.

Die 1-Imidazolyl-2-hydroxybutanderivate der allgemeinen Formel II sind noch nicht bekannt; sie sind jedoch Gegenstand einer eigenen älteren Anmeldung (vgl. Deutsche Patentanmeldung P Nr. 2918893 vom 10.5.1979) und können hergestellt werden, indem man Halogenätherketone der allgemeinen Formel III

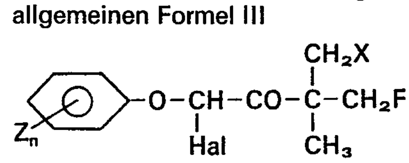 (III)

in welcher

X, Z und n die oben angegebene Bedeutung haben, und

Hal für Halogen, vorzugsweise Chlor oder Brom steht,

mit Imidazol in Gegenwart eines Säurebindemittels, wie beispielsweise Natriumcarbonat oder ein Überschuss an Imidazol, und gegebenenfalls in Gegenwart eines inerten organischen Lösungsmittels, wie beispielsweise Aceton oder Acetonitril, bei Temperaturen zwischen 60 und 120° C umsetzt, und die erhaltenen Ketoderivate nach bekannten Methoden reduziert, wie z.B. durch Umsetzung mit komplexen Hydriden, wie insbesondere Natriumborhydrid, gegebenenfalls in Gegenwart eines polaren organischen Lösungsmittels, wie beispielsweise Alkohole, bei Temperaturen zwischen 0 und 30° C; oder durch Umsetzung mit Aluminiumisopropylat in Gegenwart eines inerten organischen Lösungsmittels, wie beispielsweise Isopropanol, bei Temperaturen zwischen 20 und 120° C. Die Aufarbeitung erfolgt in üblicher Weise.

Die Halogenätherketone der allgemeinen Formel III sind noch nicht bekannt. Sie sind jedoch ebenfalls Gegenstand der oben genannten eigenen älteren Anmeldung. Sie können nach bekannten Verfahren (vgl. z.B. DE-OS Nr. 2632602) erhalten werden, indem man z.B. bekannte Phenole der allgemeinen Formel IV

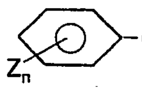 (IV)

in welcher

Z und n die oben angegebene Bedeutung haben,

mit einem Halogenketon der allgemeinen Formel V

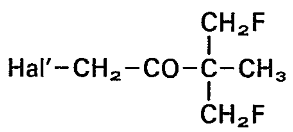 (V)

in welcher

X die oben angegebene Bedeutung hat, und

Hal' für Chlor oder Brom steht,

umsetzt. Das noch verbleibende aktive Wasserstoffatom wird anschliessend in üblicher Weise gegen Halogen ausgetauscht (vgl. auch die Herstellungsbeispiele).

Die Halogenketone der allgemeinen Formel V sind ebenfalls noch nicht bekannt und ebenfalls Gegenstand der oben genannten eigenen älteren Anmeldung. Sie können auf allgemein übliche und bekannte Weise erhalten werden, indem man Fluorderivate des 3,3-Dimethylbutan-2-ons der allgemeinen Formel VI

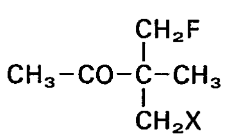 (VI)

in welcher

X die oben angegebene Bedeutung hat, mit Chlor oder Brom in Gegenwart eines inerten organischen Lösungsmittels, wie beispielsweise Äther oder chlorierte Kohlenwasserstoffe, bei Raumtemperatur versetzt (vgl. auch die Herstellungsbeispiele), oder mit üblichen Chlorierungsmitteln, wie beispielsweise Sulfurylchlorid bei 20 bis 60° C umsetzt.

Die fluorderivate des 3,3-Dimethylbutan-2-ons der allgemeine Formel sind ebenfalls nicht bekannt. Sie sind jedoch Gegenstand einer eigenen älteren Patentanmeldung (vgl. die Deutsche Patentanmeldung P Nr. 2843767 vom 6.10.1978). Man erhält die Fluorderivate des 3,3-Dimethylbutan-2-ons der allgemeinen Formel VI, wenn man Sulfonsäureester der allgemeinen Formel VII

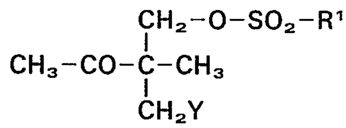 (VII)

in welcher

$R^1$ für Alkyl mit 1 bis 4 Kohlenstoffatomen, insbesondere Methyl, oder Aryl mit 6 bis 12 Kohlenstoffatomen, insbesondere Phenyl oder Tolyl, steht, und

Y für Wasserstoff oder die Gruppe $-O-SO_2-R^1$ steht,

mit Metallfluoriden, wie beispielsweise Natrium- und Kaliumfluorid, in Gegenwart eines polaren organischen Lösungsmittels, wie beispielsweise Di-, Tri- oder Tetraäthylenglykol, bei Temperaturen zwischen 80 und 250° C umsetzt (vgl. auch die Herstellungsbeispiele).

Die Sulfonsäureester der allgemeinen Formel VII sind teilweise bekannt [„J. Org. Chem." *35*, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (siehe z.B. Houben-Weyl, „Methoden der Org. Chemie", Bs. IX, S. 388 und 663, sowie die Angaben bei den Herstellungsbeispielen).

Im einzelnen seien als Beispiele für die Ausgangsstoffe der allgemeinen Formel II genannt:

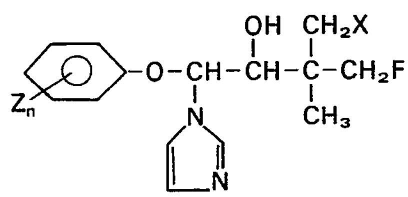 (II)

| $Z_n$ | X | $Z_n$ | X |
|---|---|---|---|
| — | H | — | F |
| 2-Cl | H | 2-Cl | F |
| 3-Cl | H | 3-Cl | F |
| 4-Cl | H | 4-Cl | F |
| 2-F | H | 2-F | F |
| 3-F | H | 3-F | F |
| 4-F | H | 4-F | F |
| 2-Br | H | 2-Br | F |
| 3-Br | H | 3-Br | F |
| 4-Br | H | 4-Br | F |
| 2,4-$Cl_2$ | H | 2,4-$Cl_2$ | F |
| 2-$CH_3$ | H | 2-$CH_3$ | F |
| 4-$CH_3$ | H | 4-$CH_3$ | F |
| 2-Cl, 4-$CH_3$ | H | 2-Cl, 4-$CH_3$ | F |

| $Z_n$ | X | $Z_n$ | X |
|---|---|---|---|
| 4-Cl, 2-$CH_3$ | H | 4-Cl, 2-$CH_3$ | F |
| 4-J | H | 4-J | F |
| 4-CN | H | 4-CN | F |
| 2-$NO_2$ | H | 2-$NO_2$ | F |
| 4-$COOCH_3$ | H | 4-$COOCH_3$ | F |
| 4-$COOC_2H_3$ | H | 4-$COOC_2H_3$ | F |
| 4-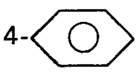 | H | 4-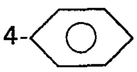 | F |
| 2-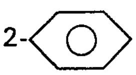 | H | 2-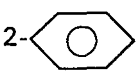 | F |
| 4-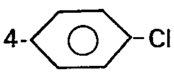 | H | 4-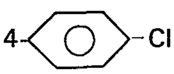 | F |

Zur Herstellung von physiologisch verträglichen Säureadditionssalzen der Verbindungen der allgemeinen Formel I kommen vorzugsweise folgende Säuren in Frage: die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäure, wie z.B. p-Toluolsulfonsäure und 1,5-Naphthalindisulfonsäure.

Die Säureadditionssalze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden, und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der allgemeinen Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe von Verbindungen der allgemeinen Formel I können in einfacher Weise nach üblichen Verfahren erhalten werden, so z.B. durch Lösen des Metallsalzes in Alkohol, z.B. Äthanol und Hinzufügen zur Verbindung der allgemeinen Formel I. Man kann Metallsalzkomplexe in bekannter Weise, z.B. durch Abfiltrieren, Isolieren und gegebenenfalls durch Umkristallisation reinigen.

Die erfindungsgemäss verwendbaren Verbindungen der allgemeinen Formel I, ihre Säureadditionssalze und Metallsalzkomplexe weisen antimikrobielle, insbesondere starke antimykotische Wirkungen auf. Sie besitzen ein sehr breites antimykotisches Wirkungsspektrum, insbesondere gegen Dermatophyten und Sprosspilze sowie biphasische Pilze, z.B. gegen *Candida*-Arten, wie *Candida albicans*, *Epidermophyton*-Arten, wie *Epidermophyton floccosum*, *Aspergillus*-Arten, wie *Aspergillus niger* und *Aspergillus fumigatus*, wie *Trichophyton*-Arten, wie *Trichophyton mentagrophytes*, *Microsporon*-Arten, wie *Microsporon felineum* sowie *Penicillium*-Arten, wie *Penicillium commune*. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch *Trichophyton mentagrophytes* und andere *Trichophyton*-Arten, *Mikrosporon*-Arten, *Epidermophyton floccosum*, Sprosspilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabletten, Dragées, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragées, Kapseln, Pillen, Granulate, Suppositorien, Lösungen, Suspensionen und Emulsionen, Pasten, Salben, Gele, Cremes, Lotions, Puder und Sprays genannt.

Tabletten, Dragées, Kapseln, Pillen und Granulate können den oder die Wirkstoffe neben den

üblichen Trägerstoffen enthalten, wie a) Füll- und Streckmittel, z.B. Stärken, Milchzucker, Rohrzukker, Glukose, Mannit und Kieselsäure, b) Bindemittel, z.B. Carboxymethylzellulose, Alginate, Gelatine, Polyvinylpyrrolidon, c) Feuchthaltemittel, z.B. Glycerin, d) Sprengmittel, z.B. Agar-Agar, Calciumcarbonat und Natriumbicarbonat, e) Lösungsverzögerer, z.B. Paraffin und f) Resorptionsbeschleuniger, z.B. quarternäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyäthylenglykole oder Gemische der unter a bis i aufgeführten Stoffe.

Die Tabletten, Dragées, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Zellulosederivate, Polyäthylenglykole, Silicone, Betonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Zellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.% der Gesamtmischung, vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

*Beispiel A*

*Antimykotische* in-vitro-*Wirksamkeit*

*Versuchsbeschreibung:*

Die *in-vitro*-Prüfung wurde im Reihenverdünnungstest mit Keiminokula von durchschnittlich 5×10 Keimen/ml Substrat durchgeführt. Als Nährmedium dienten

a) für Dermatophyten und Schimmelpilze: milieu d'épreuve de Sabouraud

b) für Hefen: Isotonic Sensitest-Broth von Oxid.

Die Bebrütungstemperatur betrug 28°C; Bebrütungsdauer war 24 h bei Hefen und 96 h bei Dermatophyten und Schimmelpilzen.

In diesen Versuchen zeigen die erfindungsgemässen Mittel gute antimykotische Wirksamkeiten.

*Beispiel B*

*Antimikrobielle* in-vivo-*Wirksamkeit (oral) bei Mäuse-Candidose*

*Versuchsbeschreibung:*

Mäuse vom Typ SPF-$CF_1$ wurden intravenös mit 1-2×$10^6$ logaritmisch wachsenden *Candida*-Zellen, die in physiologischer Kochsalzlösung suspendiert waren, infiziert. 1 h vor und 7 h nach der Infektion wurden die Tiere mit jeweils 100 mg/kg Körpergewicht der Präparate oral behandelt.

Unbehandelte Tiere starben 3 bis 6 d *post infektionem* an der Infektion. Die Überlebensrate am 6. Tag *post infektionem* betrug bei unbehandelten Kontrolltieren etwa 5%.

*Zeichenerklärung:*

+++++ = sehr gute Wirkung = ⩾90% Überlebende am 6. Tag p.i.
++++ = gute Wirkung = ⩾80% Überlebende am 6. Tag p.i.
+++ = Wirkung = ⩾60% Überlebende am 6. Tag p.i.
++ = schwache Wirkung = ⩾40% Überlebende am 6. Tag p.i.
+ = Spur Wirkung
k.W. = keine Wirkung

*Tabelle B:*

Antimykotische *in-vivo*-Wirksamkeit (oral) bei Mäuse-Candidose

| Wirkstoff | Wirkung |
|---|---|
| 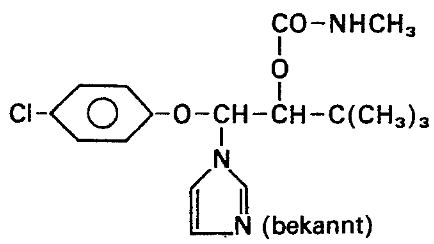 (bekannt) | k.W. |
| 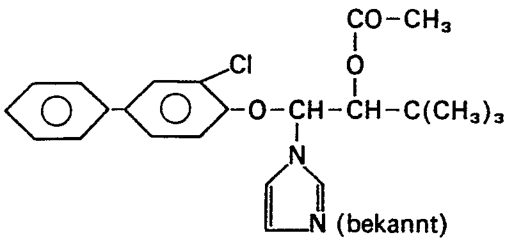 (bekannt) | k.W. |
| Verbindung aus Bsp. Nr.: | |
| 1 | ++++ |
| 2 | +++ |

*Herstellungsbeispiele*

*Beispiel 1*

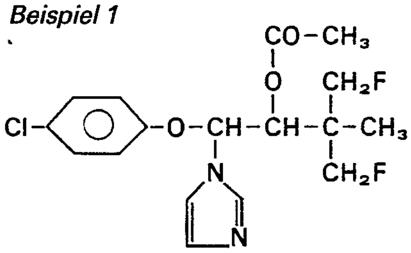

× ½

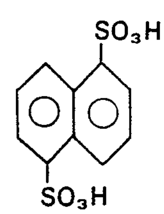

(Verfahren b)

12,2 g (0,037 mol) 3,3-Bisfluormethyl-1-(4-chlorphenoxy)-1-(imidazol-1-yl)butan-2-ol werden in 70 ml Acetanhydrid gelöst. Man lässt 16 h bei 100° C rühren, destilliert das überschüssige Acetanhydrid im Vakuum ab und nimmt den Rückstand in 300 ml Methylenchlorid auf. Die organische Phase wird zweimal mit je 800 ml Wasser gewaschen, über Natriumsulfat getrocknet und eingeengt. Der Rückstand wird mit 100 ml Aceton aufgenommen und mit einer Lösung von 9 g (0,038 mol) 1,5-Naphthalindisulfonsäure in 50 ml Aceton versetzt. Nach 2 h wird der entstandene Niederschlag abgesaugt und getrocknet. Man erhält 13 g (68% der Theorie) 2-Acetoxy-3,3-bisfluormethyl-1-(4-chlorphenoxy)-1-(imidazol-1-yl)butannaphthalindisulfonat-(1,5) vom Schmelzpunkt 221-224° C.

*Herstellung der Vorstufen*

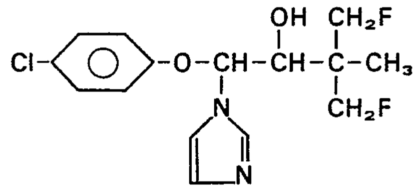

31,9 g (0,097 mol) 3,3-Bisfluormethyl-1-(4-chlorphenoxy)-1-(imidazol-1-yl)butan-2-on werden in 600 ml Methanol gelöst und bei 0 bis 5° C portionsweise mit 5,45 g (0,14 mol) Natriumborhydrid versetzt. Man lässt 15 h nachrühren, tropft 15 ml konzentrierte Salzsäure zu und verrührt 2 h bei Raumtemperatur. Danach wird das Reaktionsgemisch in 800 ml wässerige, gesättigte Natriumhydrogencarbonatlösung eingerührt. Man extrahiert mit 1000 ml Methylenchlorid, wäscht die organische Phase zweimal mit je 1000 ml Wasser, trocknet über Natriumsulfat und engt durch Abdestillieren des Lösungsmittels im Vakuum ein. Das zurückbleibende Öl wird mit Isopropyläther verrührt. Man erhält 22,2 g (67,5% der Theorie) 3,3-Bisfluormethyl-1-(4-chlorphenoxy)-1-(imidazol-1-yl)butan-2-ol vom Schmelzpunkt 137° C.

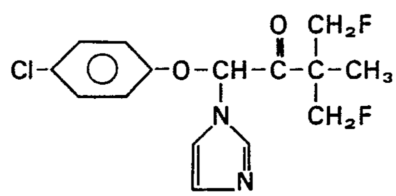

61,5 g (0,18 mol) 3,3-Bisfluormethyl-1-brom-(4-chlorphenoxy)butan-2-on werden mit 27,2 g (0,4 mol) Imidazol in 500 ml Acetonitril 4 h bei 45° C gerührt. Das Lösungsmittel wird im Wasserstrahlvakuum abdestilliert, das zurückbleibende Öl in 500 ml Methylenchlorid aufgenommen, die organische Phase zweimal mit 1000 ml Wasser gewaschen, über Natriumsulfat getrocknet und das Lösungsmittel abdestilliert. Das Öl wird in Aceton aufgenommen, mit 36 g (0,1 mol) 1,5-Naphthalindisulfonsäuretetrahydrat versetzt und der entstehende Niederschlag abgesaugt. Der Niederschlag wird mit Natriumhydrogencarbonatlösung behandelt.

Man erhält 14 g (24% der Theorie) 3,3-Bisfluormethyl-1-(4-chlorphenoxy)-1-(imidazol-1-yl)-butan-2-on als zähflüssiges Öl, das direkt weiter umgesetzt wird.

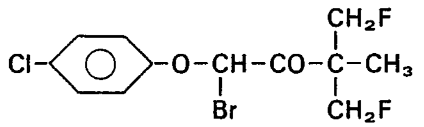

166 g (0,632 mol) 3,3-Bisfluormethyl-1-(4-chlorphenoxy)butan-2-on werden in 500 ml Methylenchlorid gelöst und bei 20 bis 30° C unter Rühren und Kühlen tropfenweise mit 100 g (0,625 mol) Brom versetzt. Es wird 2 h bei 20° C nachgerührt. Nach Abdestillieren des Lösungsmittels im Vakuum wird der Rückstand aus Petroläther kristallisiert. Man erhält 190 g (88% der Theorie) 3,3-Bisfluormethyl- 1- brom- 1- (4-chlorphenoxy)butan-2-on vom Schmelzpunkt 54-55° C.

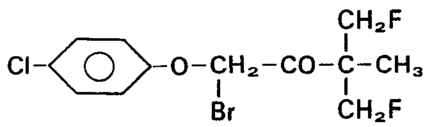

Zu einer gerührten Mischung aus 102 g (0,79 mol) p-Chlorphenol und 110 g (0,79 mol) gepulvertem Kaliumcarbonat in 500 ml Aceton werden bei 20 bis 30° C 171,2 g (0,79 mol) 3,3-Bisfluormethyl-1-brombutan-2-on zugetropft. Es wird 4 h bei 40° C nachgerührt, das anorganische Salz abfiltriert und das Filtrat eingeengt. Der Rückstand wird im Hochvakuum destilliert. Man erhält 190,5 g (90% der Theorie) 3,3-Bisfluormethyl-1-(4-chlorphenoxy)butan-2-on vom Siedepunkt 113-117° C/0,1 mmHg-Säule.

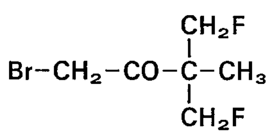

In eine Mischung aus 757 g (5,58 mol) 3,3,-Bisfluormethyl-2-butanon und 4,5 l Methylenchlorid werden bei 20 bis 30° C unter Kühlen und Rühren 903 g Brom langsam eingetropft. Die gelbliche Lösung wird noch 1 h bei 20° C nachgerührt. Nach Abdestillieren des Lösungsmittels wird der Rückstand im Vakuum destilliert. Man erhält 1030 g (86% der Theorie) 3,3-Bisfluormethyl-1-brombutan-2-on vom Siedepunkt 49-53° C/ 0,15 mmHg-Säule.

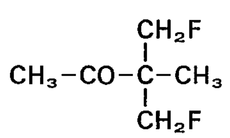

In einem Dreihalskolben mit Rührer, Tropftrichter und Liebigkühler mit gekühlter Vorlage werden 400 ml Tetraäthylenglykol und 46,4 g Kaliumfluorid (0,8 mol) vorgelegt und auf 170° C aufgeheitzt. Man legt an den Vorstoss des Liebigkühlers ein Wasserstrahlvakuum (Druck ca. 20 bis 30 mbar) an. Dann werden während 45 min 57,6 g (0,2 mol) 2-Acetyl-2-methylpropan-1,3-diolbismethansulfat, gelöst in 100 ml Tetraäthylenglykol, zugetropft. Das entstehende 3,3-Bisfluormethylbutan-2-on wird während der Reaktion in die gekühlte Vorlage abdestilliert. Nach dem Zutropfen wird noch während 1 h bei 175° C weiter destilliert.

Das aufgefangene Destillat wird anschliessend redestilliert. Man erhält 14 g (ca. 51,5% der Theorie) 3,3-Bisfluormethylbutan-2-on vom Siedepunkt 43-46° C/12 mmHg-Säule.

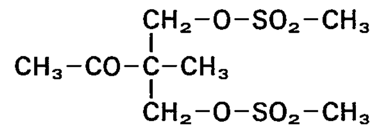

66 g (0,5 mol) 3-Oxa-2,2-bis(hydroxymethyl)-butan (zur Herstellung vgl. „Beilstein" *H 1*, E III 3306, IV 4132 und „J. Chem. Soc.", London, *1932*, 2671) werden in 300 ml 1,2-Dichloräthan gelöst, 114,5 g (1 mol) Methansulfonsäurechlorid zugetropft und bei 0-5° C 158 g (2 mol) Pyridin zugetropft. Man lässt 15 h bei Raumtemperatur nachrühren und gibt dann den Ansatz auf 600 ml Eiswasser und 100 ml konzentrierter Salzsäure. Dabei fällt ein Feststoff aus, der abgesaugt wird. Die wässerige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Äther suspendiert. Der Rückstand wird abgesaugt und mit 100 ml Äther gewaschen. Man erhält 100 g (ca. 70% der Theorie) 2-Acetyl-2-methylpropan-1,3-diolbismethansulfonat vom Schmelzpunkt 105-108° C.

*Beispiel 2*

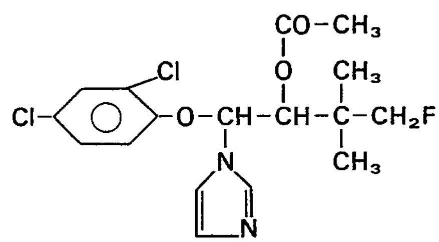

x HCl

(Verfahren b)

17,3 g (0,05 mol) 1-(2,4-Dichlorphenoxy)-3,3-dimethyl-4-fluor-1-(imidazol-1-yl)butan-2-ol werden in 100 ml Essigsäureanhydrid gelöst und nach Zugabe von 0,1 g Natriumacetat 10 h bei 100° C gerührt. Danach lässt man die Reaktionslösung auf Raumtemperatur abkühlen, rührt sie in 500 ml Wasser ein und lässt 15 h stehen. Die wässerige Phase wird zweimal mit je 200 ml Methylenchlorid extrahiert. Die vereinigten organischen Phasen werden mit je 100 ml Natriumhydrogencarbonatlösung und Wasser gewaschen, über Natriumsulfat getrocknet und im Vakuum eingeengt. Der Rückstand wird in 100 ml Äther aufgenommen, mit 20 ml ätherischer Salzsäure versetzt, das Lösungsmittel im Vakuum abdestilliert und der Rückstand mit 50 ml Äther verrührt. Man erhält 15,3 g (72% der Theorie) 2-Acetoxy-1-(2,4-dichlorphenoxy)-3,3-dimethyl-4-fluor-1-(imidazol-1-yl)butanhydrochlorid vom Schmelzpunkt 198-200° C.

*Herstellung der Vorstufen*

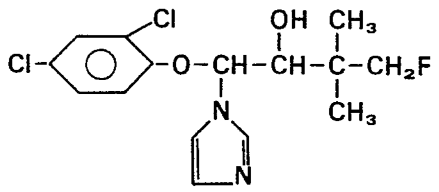

x HCl

44 g (0,1275 mol) 1-(2,4-Dichlorphenoxy)-3,3-dimethyl-4-fluor-1-(imidazol-1-yl)-2-butanon werden in 300 ml Methanol gelöst und bei 0 bis 5° C mit 6,3 g Natriumborhydrid versetzt. Man lässt 15 h bei Raumtemperatur nachrühren, tropft 60 ml konzentrierte Salzsäure unter Eiskühlung zu, verrührt 10 h bei Raumtemperatur und destilliert das Lösungsmittel im Wasserstrahlvakuum ab. Der Rückstand wird in 250 ml Methylenchlorid aufgenommen, in 500 ml wässerige, gesättigte Natriumhydrogencarbonatlösung eingerührt, die Methylenchloridphase abgetrennt, dreimal mit je 100 ml Wasser gewaschen, die organische Phase über Natriumsulfat getrocknet und das Lösungsmittel abdestilliert. Das zurückbleibende Öl wird in 200 ml Äther aufgenommen, 50 ml ätherische Salzsäure zugegeben und das Lösungsmittel abdestilliert. Man erhält 28,2 g (58% der Theorie) 1-(2,4- Dichlorphenoxy)- 3,3- dimethyl- 4- fluor-1- (imidazol- 1- yl)- 2- butanolhydrochlorid vom Schmelzpunkt 184-210° C.

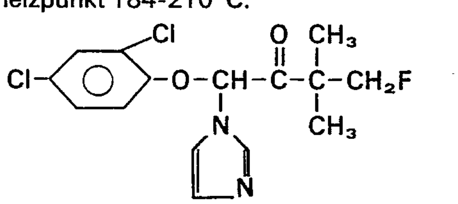

157,5 g (0,44 mol) 1-Brom-1-(2,4-dichlorphenoxy)-3,3-dimehtyl-4-fluor-2-butanon werden in 500 ml Acetonitril gelöst und zu einer Lösung von 109 g (1,6 mol) Imidazol in 600 ml Acetonitril getropft. Man erhitzt 10 h unter Rückfluss. Danach wird das Lösungsmittel im Wasserstrahlvakuum abdestilliert, der Rückstand in 1000 ml Methylenchlorid aufgenommen und dreimal mit je 500 ml Wasser gewaschen. Die organische Phase wird über Natriumsulfat getrocknet, filtriert und im Wasserstrahlvakuum durch Abdestillieren des Lösungsmittels eingeengt. Der Rückstand wird in 500 ml Äthanol gelöst, mit je 40 ml konzentrierter Salzsäure versetzt und das Lösungsmittel im Wasserstrahlvakuum abdestilliert. Der Rückstand wird mit 500 ml Äther verrührt, wobei es zur Kristallisation kommt. Man erhält 66 g (39,3% der Theorie) 1- (2,4- Dichlorphenoxy)- 3,3- dimethyl- 4-fluor- 1- (imidazol- 1- yl)- 2- butanonhydrochlorid vom Schmelzpunkt 91-110° C.

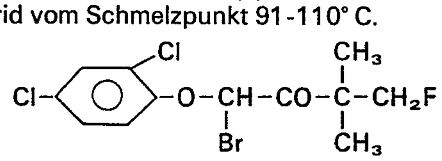

199,5 g (0,71 mol) 1-(2,4-Dichlorphenoxy)-3,3-dimethyl-4-fluor-2-butanon werden in 500 ml Chloroform gelöst und bei 20 bis 30° C unter Rühren und Kühlen tropfenweise mit 114 g (0,71 mol) Brom versetzt. Es wird 2 h bei 20° C nachgerührt, vorsichtig mit 200 ml Wasser versetzt, die Chloroformphase mehrmals mit Eiswasser gewaschen und über Natriumsulfat getrocknet. Nach Abdestillieren des Lösungsmittels im Vakuum erhält man 205,2 g (78% der Theorie) 1-Brom- 1- (2,4- dichlorphenoxy)- 3,3- dimethyl-4- fluor- 2- butanon als Öl, das direkt weiter umgesetzt wird.

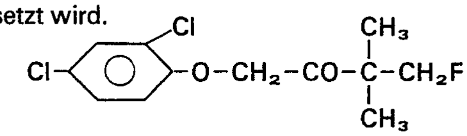

Zu einer gerührten Mischung aus 129 g (0,79 mol) 2,4-Dichlorphenol und 110 g (0,79 mol) gepulvertem Kaliumcarbonat in 500 ml Aceton werden unter Kühlen bei 20 bis 30° C 157 g (0,79 mol) 1-Brom-3,3-dimethyl-4-fluor-2-butanon zugetropft. Es wird 2 h bei 20° C nachgerührt, das anorganische Salz abfiltriert und das Filtrat eingeengt. Man erhält 199,3 g (90% der Theorie) 1-(2,4-Dichlorphenoxy)-3,3-dimethyl-4-fluor-2-butanon als Öl, das direkt weiter umgesetzt wird.

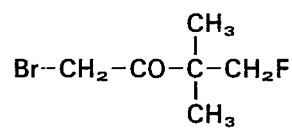

In eine Mischung aus 354 g (3 mol) 3,3-Dimethyl-4-fluor-2-butanon und 2000 ml Äther werden bei 20 bis 30° C unter Kühlen und Rühren 480 g Brom langsam eingetropft. Die gelbliche Lösung wird noch 1 h bei 20° C nachgerührt und anschliessend vorsichtig mit 500 ml Wasser versetzt. Die Ätherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80-90° C/11 mmHg-Säule.

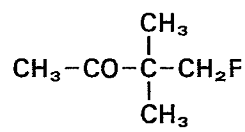

Zu der in einem Dreihalsrührkolben mit absteigendem Kühler befindlichen Suspension von 23,2 g (0,4 mol) trockenem Kaliumfluorid in 400 ml destilliertem Tetraäthylenglykol werden bei 160° C und 20 mbar 38,8 g (0,2 mol) 2,2-Dimethyl-2-oxobutylmethansulfonat im Verlauf von 2 h zugetropft und 2 weitere h nachgerührt. An einem absteigenden Kondensator und in einer nachgeschalteten Tiefkühlfalle wird das herausdestillierte Reaktionsprodukt kondensiert und gesammelt. Man erhält 20,9 g (89% der Theorie) 3,3-Dimethyl-4-fluor-2-butanon, vom Siedepunkt 130-134° C.

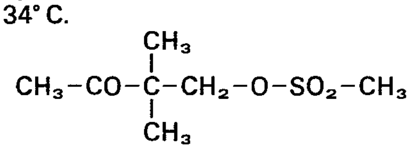

232 g (2 mol) 3,3-Dimethyl-4-hydroxy-2-butanon (z.Herstellung vgl. „Beilstein" *H 1*, E III 3239, IV 4030 und „Bull. Soc. Chim.", France, *1964*, 2849) werden in 700 ml absolutem Pyridin bei 0 bis 5° C mit 229 g (2 mol) Methansulfochlorid umgesetzt. Nach 12 h Stehen bei 20° C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxobutylmethansulfonat vom Siedepunkt 106-120° C/0,12 mmHg-Säule.

In entsprechender Weise und gemäss den Verfahren a bis b werden die nachfolgenden Beispiele der allgemeinen Formel I

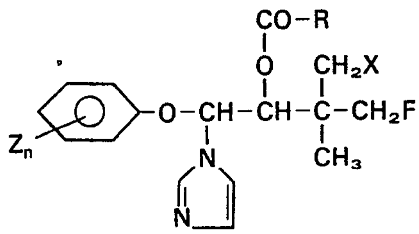

(I)

erhalten:

| Beispiel Nr. | $Z_n$ | R | X | Schmelzpunkt (°C) |
|---|---|---|---|---|
| 3 | 4-F | $-CH_3$ | F | 200-210 (×½ NDS) |
| 4 | 2,4-$Cl_2$ | $-CH_3$ | F | 195-205 (×½ NDS) |
| 5 | 4-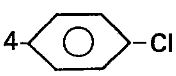-Cl | $-CH_3$ | F | 256 (×½ NDS) |
| 6 | 2,4-$Cl_2$ | -NH-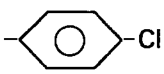-Cl | F | 142-44 |
| 7 | 4-Cl | -NH-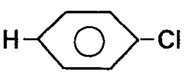-Cl | F | 146-48 |
| 8 | 4-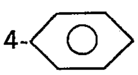 | -NH-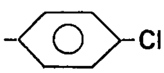-Cl | F | 124 |
| 9 | 2,4-$Cl_2$ | 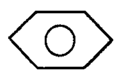 | F | 140-42 |
| 10 | 4-Cl | 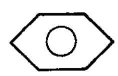 | F | 58-60 |
| 11 | 4-Cl | $-CH_2CH_2CH_2Cl$ | F | 225-28 (×½ NDS) |
| 12 | 4-Cl | 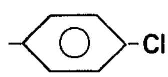-Cl | F | >260 (×½ NDS) |
| 13 | 2,4-$Cl_2$ | 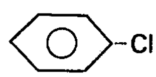-Cl | F | >260 (×½ NDS) |
| 14 | 2,4-$Cl_2$ | $-CH_2CH_2CH_2Cl$ | F | 255-60 (×½ NDS) |
| 15 | 2,4-$Cl_2$ | $-CH_2OC_2H_5$ | F | 222-24 (×½ NDS) |
| 16 | 4-Cl | $-CH_2OC_2H_5$ | F | 225-27 (×½ NDS) |
| 17 | 4-Cl | $-NHCH_3$ | F | 121-27 |
| 18 | 2,4-$Cl_2$ | $-NHCH_3$ | F | 250 (×½ NDS) |
| 19 | 2,4-$Cl_2$ | $-CHCl_2$ | F | 215-25 (×½ NDS) |

**Patentansprüche**

1. Antimykotisches Mittel, gekennzeichnet durch einen Gehalt an mindestens einem acylierten Imidazolyl-γ-fluorpinakolylderivat der allgemeinen Formel (I)

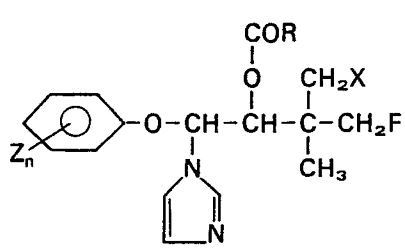

(I)

in welcher

R für Methyl, Äthyl, Isobutyl, Chlormethyl, Dichlormethyl, Chloräthyl, Chlorpropyl, Methacryl, Cyclohexyl, gegebenenfalls einfach oder mehrfach durch Chlor, Brom, Methyl oder Methoxy substituiertes Phenyl, Benzyl oder Phenoxymethyl, für Methoxy, Äthoxy, Isopropoxy, Butoxy oder Isobutoxy, Methyl- oder Äthylamino, Dimethylamino, Methoxycarbonylamino, Äthoxycarbonylamino oder Methoxymethylamino steht,

X für Wasserstoff oder Fluor steht,

Z für Chlor, Brom, Methyl, Äthyl, Cyclohexyl, Methoxy, Methylthio, Trifluormethyl, Methoxycarbonyl, Cyano, Nitro oder gegebenenfalls durch Chlor substituiertes Phenyl, Benzyl oder Phenoxy steht, und

n für 0, 1 oder 2 steht,

und/oder deren physiologisch verträglichen Säureadditionssalzen und Metallsalzkomplexen.

2. Antimykotisches Mittel nach Anspruch 1, gekennzeichnet durch einen Gehalt an 2-Acetoxy-3,3-bisfluormethyl-1-(4-chlorphenoxy)-1-(imidazol-1-yl)butan.

3. Antimykotisches Mittel nach Anspruch 1, gekennzeichnet durch einen Gehalt an 2-Acetoxy-3,3-bisfluormethyl-1-(4-chlorphenoxy)-1-(imidazol-1-yl)butannaphthalindisulfonat-(1,5).

4. Antimykotisches Mittel nach Anspruch 1, gekennzeichnet durch einen Gehalt an 2-Acetoxy-1-(2,4-dichlorphenoxy)-3,3-dimethyl-4-fluor-1-(imidazol-1-yl)butan.

5. Antimykotisches Mittel nach Anspruch 1, gekennzeichnet durch einen Gehalt an 2-Acetoxy-1-(2,4-dichlorphenoxy)-3,3-dimethyl-4-fluor-1-(imidazol-1-yl)butanhydrochlorid.

6. Verfahren zur Herstellung eines antimykotischen Mittels, dadurch gekennzeichnet, dass man mindestens ein acyliertes Imidazolyl-γ-fluorpinakolylderivat gemäss der allgemeinen Formel (I) in Anspruch 1 mit inerten, nichttoxischen, pharmazeutisch geeigneten Trägerstoffen vermischt.

## Claims

1. Antimycotic agent, characterized in that it contains at least one acylated imidazolyl-γ-fluoropinacolyl derivative of the general formula

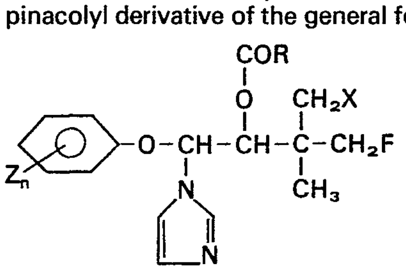

(I)

in which

R represents methyl, ethyl, isobutyl, chloromethyl, dichlormethyl, chloroethyl, chloropropyl, methacryl or cyclohexyl, or phenyl, benzyl or phenoxymethyl which is optionally monosubstituted or polysubstituted by chlorine, bromine methyl or methoxy, or methoxy, ethoxy, isopropoxy, butoxy, isobutoxy, methyl- or ethylamino, dimethylamino, methoxycarbonylamino, ethoxycarbonylamino or methoxymethylamino,

X represents hydrogen or fluorine,

Z represents chlorine, bromine, methyl, ethyl, cyclohexyl, methoxy, methylthio, trifluoromethyl, methoxycarbonyl, cyano or nitro, or phenyl, benzyl or phenoxy which is optionally substituted by chlorine, and

n represents 0, 1 or 2,

and/or physiologically acceptable acid addition salts and metal salt complexes thereof.

2. Antimycotic agent according to claim 1, characterized in that it contains 2-acetoxy-3,3-bis fluoromethyl-1-(4-chlorophenoxy)-1-(imidazol-1-yl)butane.

3. Antimycotic agent according to claim 1, characterized in that it contains 2-acetoxy-3,3-bisfluoromethyl - 1 - (4 - chlorophenoxy) - 1 - (imidazol - 1 - yl)butane naphthalene - 1,5-disulphonate.

4. Antimycotic agent according to claim 1, characterized in that it contains 2-acetoxy-1-(2,4-dichlorophenoxy)-3,3-dimethyl-4-fluoro-1-(imidazol-1-yl)butane.

5. Antimycotic agent according to claim 1, characterized in that it contains 2-acetoxy-1-(2,4-dichlorophenoxy)-3,3-dimethyl-4-fluoro-1-(imidazol-1-yl)butane hydrochloride.

6. Process for the preparation of an antimycotic agent, characterized in that at least one acylated imidazolyl-γ-fluoropinacolyl derivate of the general formula (I) in claim 1 is mixed with inert, non-toxic, pharmaceutically suitable excipients.

## Revendications

1. Agent antimycotique, caractérisé en ce qu'il contient au moins un dérivé d'imidazolyl-γ-fluoropinacolyle de formule générale

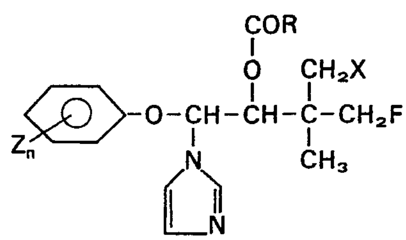

(I)

dans laquelle

R représente un reste méthyle, éthyle, isobutyle, chlorométhyle, dichlorométhyle, chloroéthyle, chloropropyle, méthacryle, cyclohexyle, un reste phényle, benzyle ou phénoxyméthyle éventuellement mono- ou polysubstitué par le chlore, le brome ou un groupe méthyle ou méthoxy, ou un groupe méthoxy, éthoxy, isopropoxy, butoxy ou isobutoxy, méthyl- ou éthylamino, diméthylamino, méthoxycarbonylamino, éthoxycarbonylamino ou méthoxyméthylamino,

X représente l'hydrogène ou le fluor,

Z représente le chlore, le brome ou un groupe méthyle, éthyle, cyclohexyle, méthoxy, méthylthio, trifluorométhyle, méthoxycarbonyle, cyano ou nitro ou un groupe phényle, benzyle ou

phénoxy éventuellement substitué par le chlore, et
n est égal à 0, 1 ou 2,
et/ou leurs sels d'addition d'acides et leurs complexes de sels métalliques physiologiquement compatibles.

2. Agent antimycotique selon la revendication 1, caractérisé en ce qu'il contient du 2-acétoxy-3,3 - bisfluorométhyl - 1 - (4 - chlorophénoxy) - 1 - (imidazole-1-yl)butane.

3. Agent antimycotique selon la revendication 1, caractérisé en ce qu'il contient du naphtalènedisulfonate-1,5 de 2-acétoxy-3,3-bisfluorométhyl - 1 - (4 - chlorophénoxy) - 1 - (imidazole-1-yl)butane.

4. Agent antimycotique selon la revendication 1, caractérisé en ce qu'il contient du 2-acétoxy-1-(2,4 - dichlorophénoxy) - 3,3 - diméthyl - 4 - fluoro-1-(imidazole-1-yl)butane.

5. Agent antimycotique selon la revendication 1, caractérisé en ce qu'il contient du chlorhydrate de 2 - acétoxy - 1 - (2,4 - dichlorophénoxy) - 3,3 - diméthyl-4-fluoro-1-(imidazole-1-yl)butane.

6. Procédé pour la fabrication d'un agent antimycotique, caractérisé en ce que l'on mélange au moins un dérivé acylé d'imidazolyl-γ-fluoropinacolyle selon la formule générale (I) dans la revendication 1, avec des supports appropriés non toxiques, pharmaceutiquement acceptables.